# EUROPEAN PATENT APPLICATION

(11) **EP 2 283 734 A1**
(43) Date of publication of application: **16.02.2011**
(21) Application number: 09010330.0
(22) Date of filing: 11.08.2009
(51) Int. Cl.: A23K 1/00, C11B 13/00, C10L 1/08, C12N 1/12, C12R 1/89, C02F 3/32

(54) **Process to obtain fat and/or oil from industrial waste to use them as raw materials for the production of fuels**

(71) Applicant: Biodiesel del Plata S.A., Buenos Aires (AR)
(72) Inventor: Piwko, Jorge Alejandro, Buenos Aires, (AR)
(74) Representative: Temino Ceniceros, Ignacio

(57) **Abstract**

Process to obtain fat and/or oil from industrial waste to use them as raw materials for the production of fuels, which includes a prior stage consisting in the collection of industrial waste, and waste from effluent treatment industries; a first stage consisting in the acidification of industrial waste, a second stage where most organic matter is separated from fluids by way of centrifugation; a third stage consisting in the extraction of fat and fatty acids; a fourth stage, consisting in the separation fat, fatty acids and solvent from the remaining organic matter; a fifth stage, where the mixture -including fat, fatty acids and solvent- is heated; a sixth stage, where the nonfat organic matter and the fatty acids are put into a pool with microalgae; and a seventh stage, which consists in verifying the acidity of the mixture of fat and fatty acids.

## Description

In order that this invention may be understandable and easily carried into practice, the following paragraphs include a detailed description of a referred mode of embodiment with reference to the accompanying drawings, which are only for the purpose of illustration and are not to be construed as limiting the scope of the invention in any manner. Equivalents may be substituted for elements thereof without departing from the spirit of the invention as described in this disclosure.

PRIOR ART. PURPOSE: There is currently a debate over whether it is convenient or not to assign an increasing amount of land for the production of vegetal species which may be processed to obtain fuels.

On the one hand, pollution resulting from the use of fossil fuels, together with their growing demand and decreasing supply, has accelerated the search for alternatives which make it possible to produce the daily volume of energy that we require.

Eolic, hydraulic, and solar generation have been or are about to be studied and implemented as possible ways of obtaining energy, together with how to take advantage of energy deriving from tide amplitude, hydrogen, etc.

In recent years, the manufacture of fuels from alcohol obtained from crops, such as sugar canes, has enjoyed a boom, just like the so-called "gasohol" in the Federative Republic of Brazil or the manufacture of fuels from oil extracted from corn or soybeans, which are known as "biodiesel".

The truth is that, in view of the current use of fuels and their known reserves, it may be affirmed that there will be enough fossil fuels until the middle of this century.

The gravity of the situation has led to increasing efforts to obtain fuels from crops, yet this entails a number of problems which are as serious as or even more severe than pollution.

Indeed, if forest is destroyed to raise the production of the so-called biofuels by increasing the arable land, the result is just the opposite to that desired, since the ability of woods and forests to clean the air is higher than that of the crops they are replaced with.

On the other hand, the competition for arable land has sparked off a debate over whether to prioritize fuels or foodstuffs, which will be certainly one of the main confrontations of the 21 st Century.

The 21st Century must be considered as the Miracle Century. The technological improvements in every field have been significantly higher than those achieved at any other time in the history of mankind and have led to important changes in the quality of life.

The key factor in this massive increase was the possibility of managing without the energy obtained almost exclusively from the sun and having new sources of energy at our disposal, mainly from oil and its derivatives.

The uncontrolled growth resulting from this change gave rise to undesired consequences, such as the greenhouse effect (global warming) and environmental pollution.

Gasoil constitutes the fuel which is most consumed in the world and grows at a cumulative annual rate of 2.5%. With a global consumption of around 1,3 billion cubic meters per year, the United States, Europe, China and Japan represent slightly over 50% of consumption.

The scenario foreseen by the Argentine Institute for Fanning Technology (INTA) shows that the global consumption for 2018 will amount to about 1,526 billion cubic meters of gasoil per year.

The so-called biodiesel is, among other alternative fuels, the favorite one since, if used as a natural additive to gasoil, it makes it possible to reduce environmental pollution and to control the greenhouse effect, which is caused by fossil fuel consumption.

The great environmental problem of the Earth is that the biosphere is sick. The environmental balance was broken, as demonstrated by the Earth through the rage of natural disasters. This causes the ecosystem to collapse and jeopardizes a sustainable future.

Warnings began with minor changes, which were noticed by man only a few decades ago and acknowledged by governments even later.

The Stern report and the fourth report by the IPCC (Intergovernmental Panel on Climate Change) have ratified this serious issue, as well as its costs and social and economic consequences. Global warming, which could be of up to 6 degrees Celsius, could take up to 15% of the countries' GDP. Even the best conventional economic growth rates will be surpassed by the harm to societies resulting from a great and fiercer weather variation.

The above mentioned fourth report by the IPCC has made it clear that weather change is mainly caused by man, and that fossil fuel consumption and deforestation constitute the first two causes. An American inhabitant issues 10 times more greenhouse effect gases than a Chinese or Indian inhabitant. However, the substantial improvement in the quality of life of the inhabitants of these giant Asian countries causes a quick leveling of the above disparity. Therefore, unless countries decide to choose an alternative and novel energy development, consequences will be increasingly worse.

Weather variations are having a growing impact on people and their customs, as observed in certain European countries and some American states. In view of the seriousness of the problem and its consequences, the Kyoto Protocol represents a timid attempt and the European Union is proposing "2 degrees less" for 2020. This is an arduous, complex task and it will require radical decisions both in developed and developing countries, which have stated so far that they will not do anything unless developed countries reduce their emissions.

The purpose of this invention is to provide a process which makes it possible to obtain oil and fat from which fuels may be produced, by using raw materials which do not derive from oilseeds.

This allows arable land to be exclusively assigned to the production of foodstuffs, thus making it unnecessary to deforest or decide between food and fuels.

The process which will be described further below was developed for such purpose. Such process makes use of the wrongly-called "fat" and "mud" from production waste, bad batches, sewage effluents from the dairy and meet-processing industries, effluent treatment, etc.

The fat or mud is obtained by processing industrial waste through DAF (Dissolved Air Flotation) separators which cause the detachment of emulsified drops by pulling them through small air bubbles. There are other known methods, and technological improvement will surely create new processes with similar results in the future.

For the above purpose, the certain additives such as iron, calcium oxide, hydrogen peroxide, and polymers are added to the waste to facilitate separation. Then, air is added at a high pressure in order to increase the dissolved air content.

When liquid waste enters the DAF unit, pressure is released and the dissolved air becomes separated from the solution, forming bubbles around any solid remaining in the solution or the interfaces of the scattered hydrocarbon drops.

In this manner, the scattered hydrocarbon drops attached to the air bubbles are pulled upwards and float where they will be subsequently removed.

Nowadays, industrial waste which will be used in the process herein disclosed is being delivered by industries to specialized companies for its final disposal in exchange for a service fee.

The process indicated in this disclosure converts the above waste into oil which may be processed in biodiesel refining operations. Such oil may be processed in almost any existing biodiesel refinery, it being unnecessary to install any additional facility to those already existing.

DRAWINGS: The accompanying figure is a block diagram of the necessary steps to conduct the process herein disclosed.

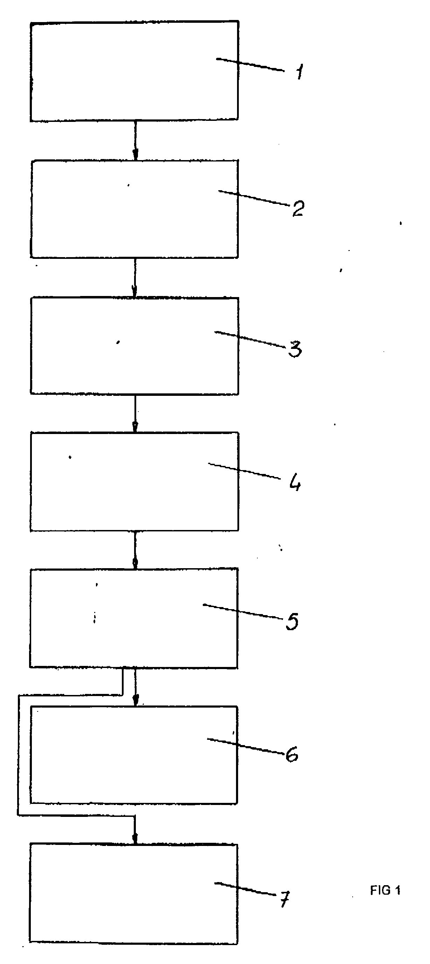

REFERENCES: In the figure, wherein like reference characters indicate like or corresponding parts, Figure 1 is the first stage; Figure 2, the second stage; Figure 3, the third stage; Figure 4, the fourth stage; Figure 5, the fifth stage; Figure 6, the sixth stage; and Figure 7, the seventh stage.

DESCRIPTION: This invention basically consists in a process made up of Stage 1: Acidification; Stage 2: Centrifugation; Stage 3: Fat and Fatty Acid Extraction; Stage 4: Separation; Stage 5: Distillation; Stage 6: Treatment of Non-Fat Organic Matter in a Pool with Microalgae; and Stage 7: Esterification or Transesterification, based on the acidity level.

OPERATION: Having the different components that explain the nature of the invention been established, this description is now complemented with the functional and operational explanation of its parts and the result they bring about.

In order to develop a process to obtain fat and/or oil from industrial waste to use them as raw materials for the production of fuels, at a prior stage, industrial waste, such as production waste, bad batches, sewage effluents from the dairy and meet-processing industries, waste from effluent treatment industries, etc., is collected.

This process is made up of Stage 1, where the abovedescribed industrial waste is acidified and heated, and Stage 2, where organic matter is separated from fluids by centrifugation.

The process also includes a Stage 3, where solvent is added in order to extract fat and fatty acids, and a Stage 4, which entails the removal of the fat, fatty acids and solvent from the remaining organic matter by way of centrifugation.

There is also a Stage 5, which consists in extracting the solvent by distillation; a Stage 6, where nonfat organic matter is treated in a pool with microalgae, creating a biomass which may be used as animal food, to extract algae oil or for other purposes; and a Stage 7, where the acidity of the fluid is verified and, if appropriate, adjusted in order to send it to the refinery to obtain a biodiesel-type fuel.

For the purpose of allowing the above sequence of stages to be reproduced and to obtain oil with suitable characteristics to allow its refining and the production of fuel, in order to acidify the industrial waste, at Stage 1, either hydrochloric, sulphuric or phosphoric acid is added in an amount sufficient to cause the pH of the waste to reach a value between 1.8 and 2.4.

At Stage 1, industrial waste is heated at a temperature ranging between 44 and 55 degrees Celsius.

The purpose of acidification and heating is to eliminate the highest volume of bacteria possible which the mixture may contain, taking into consideration the fact that it is made from expired, bad, wrongly-manufactured products; production waste and sewage waste, usually produced by certain industries.

Acidification and heating also make it possible to break any soap molecule remaining from washing water by releasing its fatty acids.

At Stage 2, organic matter is separated from fluids by way of centrifugation at a speed between 1800 and 2500 rpm during a period between 25 and 35 minutes.

At Stage 3, in order to extract fat and fatty acids, a common prior art method is used, i.e. the addition of a nonpolar solvent such as hexane or the like.

This solvent is added in an amount to be determined based on the type of waste but which is estimated to range between a minimum of 20/80 and a maximum of 40/60.

For the purpose of optimizing the results, at Stage 3, the temperature of the waste remains unaltered, and such waste is subject to slow shaking between 25 and 35 minutes.

As previously stated, Stage 4 consists in the separation of fat, fatty acids and solvent from the remaining organic matter by centrifugation. Centrifugation is conducted between 25 and 35 minutes at a speed between 1800 and 2200 rpm.

At Stage 5, solvent is recovered by distillation. For such purpose, the mixture containing the fat, fatty acids and the solvent itself is mixed at a temperature between 50 and 60 degrees Celsius and kept at a pressure between 180 and 200 mmHg between 50 and 75 minutes.

The pressure level indicated above makes it possible to reduce the distillation temperature and carry out a subsequent condensation of the solvent.

At this stage of the process, there is a mixture of fat and fatty acids, and a mixture of nonfat organic matter which go through different stages.

Thus, at Stage 6, the mixture of nonfat organic matter is put into a pool with microalgae in order to create a biomass which may serve different purposes, such as animal food or the production of algae oil.

Even though the use of microalgae for the treatment of effluents constitutes part of the prior art, the inventor has determined that, at this stage, it is convenient for the process to be conducted at room temperature.

Although any type of microalgae regularly used for this purpose may be chosen, the inventor has obtained better results with the Botryococcus specie.

Furthermore, , at Stage 7, the mixture of fat and fatty acids resulting from the distillation process conducted at Stage 5 is subject to acidity control and, in its case, adjustment in order to send the resulting mixture to a refinery to obtain a biodiesel-type fuel.

Acidity control will determine if the acidity level of the mixture of fat and fatty acids is higher or lower than 2%. If lower, it will be subject to transesterification at any biodiesel refinery, just like any other vegetable oil used for biodiesel production.

If higher, prior esterification will be conducted to make the mixture suitable for a subsequent transesterification, which will lead to the production of fuel.

At Stage 7, the acidity level must be 2%, as measured through a titration analytical process which, by using a logarithmic expression relating to the equilibrium concentration of acids, would result in a pH of about 5.5.

In accordance with a preferred mode of embodiment, phosphoric acid is used at the first stage of the process.

In this manner, one of the constructive possibilities that provide an explanation of the invention and the manner in which it works has been described. The documentation is supplemented with the synthesis of the invention contained in the claims, which are attached hereto.

Having described and determined the nature of the invention, its scope and the manner in which its fundamental concept can be put into practice, the following is declared as invention and exclusive property:

## Claims

1. Process to obtain fat and/or oil from industrial waste to use them as raw materials for the production of fuels, which includes a prior stage consisting in the collection of industrial waste, such as production waste, bad batches, sewage effluents from the dairy and meet-processing industry, and waste from effluent treatment industries; a third stage consisting in the extraction of fat and fatty acids by adding a nonpolar solvent; a sixth stage, where the nonfat organic matter and the fatty acids are put into a pool with microalgae; and is **characterized by** also including a first stage, which consists in the acidification of industrial waste by adding either hydrochloric, sulphuric or phosphoric acid in an amount sufficient to cause the pH of the waste to reach a value between 1.8 and 2.4., as well as in heating waste at a temperature between 44 and 55 degrees Celsius; a second stage, where most organic matter is separated from fluids by way of centrifugation at a speed between 1800 and 2500 rpm for a period between 25 and 35 minutes; a third stage, where nonpolar solvent is added in an amount ranging between a minimum of 20/80 and a maximum of 40/60 and the mixture is subject to slow shaking for a period between 25 and 35 minutes; a fourth stage, consisting in the separation fat, fatty acids and solvent from the remaining organic matter by way of centrifugation at a speed between 1800 and 2200 rpm for a period between 25 and 35 minutes; a fifth stage, where the mixture - including fat, fatty acids and solvent- is heated at a temperature between 50 and 60 degrees Celsius and at a pressure between 180 and 200 mmHg for a period between 50 and 75 minutes, and the solvent is recovered by distillation; and a seventh stage, which consists in verifying the acidity of the mixture of fat and fatty acids resulting from distillation and includes, if applicable, transesterification, if lower than 2%, and prior esterification, if higher than such value.

2. In accordance with Claim No. 1, this process is **characterized in that** the acid chosen at Stage 1 is phosphoric acid.

3. In accordance with Claim No. 1, this process is **characterized in that**, at Stage 3, hexane is the nonpolar solvent which is used.

4. In accordance with Claim No. 1, this process is **characterized in that**, at Stage 6, the microalgae used belong to the Botryococcus specie.

5. In accordance with Claim No. 1, this process is **characterized in that**, at Stage 6, the organic matter where the microalgae act is at room temperature.
